(19) **Europäisches Patentamt / European Patent Office / Office européen des brevets**

(11) **EP 3 879 582 B1**

(12) # EUROPEAN PATENT SPECIFICATION

(45) Date of publication and mention
of the grant of the patent:
**23.07.2025 Bulletin 2025/30**

(21) Application number: **19881753.8**

(22) Date of filing: **28.10.2019**

(51) International Patent Classification (IPC):
***A61B 5/00*** *(2006.01)*

(52) Cooperative Patent Classification (CPC):
**A61B 5/263; A61B 5/293; A61B 5/367; A61B 5/37;**
A61B 2562/0217; A61B 2562/046

(86) International application number:
**PCT/ES2019/070728**

(87) International publication number:
**WO 2020/094898 (14.05.2020 Gazette 2020/20)**

(54) **SYSTEM OF GRAPHENE TRANSISTORS FOR MEASURING ELECTROPHYSIOLOGICAL SIGNALS**

SYSTEM AUS GRAPHENTRANSISTOREN ZUR MESSUNG ELEKTROPHYSIOLOGISCHER SIGNALE

SYSTÈME À TRANSISTORS EN GRAPHÈNE POUR MESURER DES SIGNAUX ÉLECTROPHYSIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.11.2018 ES 201831068**

(43) Date of publication of application:
**15.09.2021 Bulletin 2021/37**

(73) Proprietors:
• **Consejo Superior de Investigaciones Cientificas**
**28006 Madrid (ES)**
• **Consorcio Centro de Investigación Biomédica en Red**
**M.P.**
**28029 Madrid (ES)**
• **Institució Catalana de Recerca i Estudis Avançats**
**(ICREA)**
**08010 Barcelona (ES)**
• **Fundació Institut Català de Nanociència i Nanotecnologia (ICN2)**
**08193 Bellaterra (ES)**
• **Instituto de Investigaciones Biomédicas August Pi Sunyer (IDIBAPS)**
**08036 Barcelona (ES)**

(72) Inventors:
• **GUIMERÁ BRUNET, Antón**
**08193 Cerdanyola del Vallés, Bellaterra, BARCELONA (ES)**
• **MASVIDAL CODINA, Eduard**
**08193 Cerdanyola del Vallès (Bellaterra) BARCELONA (ES)**
• **VILLA SANZ, Rosa**
**08193 Cerdanyola del Vallès (Bellaterra) BARCELONA (ES)**
• **GARRIDO ARIZA, José Antonio**
**08193 Bellaterra, BARCELONA (ES)**
• **ILLA VILA, Xavier**
**28029 Madrid (ES)**
• **SANCHEZ VIVES, María Victoria**
**08036 BARCELONA (ES)**

(74) Representative: **Witte, Weller & Partner Patentanwälte mbB**
**Postfach 10 54 62**
**70047 Stuttgart (DE)**

(56) References cited:
**WO-A1-2012/126003     GB-A- 2 471 672**

**(Cont. next page)**

- BLASCHKE BENNO M ET AL: "Mapping brain activity with flexible graphene micro-transistors", 2D MATERIALS, vol. 4, no. 2, 24 February 2017 (2017-02-24), pages 025040, XP055937699, Retrieved from the Internet <URL:https://iopscience.iop.org/article/10.1088/2053-1583/aa5eff> DOI: 10.1088/2053-1583/aa5eff
- CHENG JI ET AL: "Flexible Solution-Gated Graphene Field Effect Transistor for Electrophysiological Recording", JOURNAL OF MICROELECTROMECHANICAL SYSTEMS, IEEE SERVICE CENTER, US, vol. 23, no. 6, 1 December 2014 (2014-12-01), pages 1311 - 1317, XP011565564, ISSN: 1057-7157, [retrieved on 20141125], DOI: 10.1109/JMEMS.2014.2312714
- LUCAS H HESS ET AL: "Graphene Transistors for Bioelectronics", PROCEEDINGS OF THE IEEE, IEEE. NEW YORK, US, vol. 101, no. 7, 1 July 2013 (2013-07-01), pages 1780 - 1792, XP011515028, ISSN: 0018-9219, DOI: 10.1109/JPROC.2013.2261031

- FAN XUGE: "Integration of graphene into MEMS and NEMS for sensing applications", 1 January 2018 (2018-01-01), XP055937464, ISBN: 978-91-7-729803-8, Retrieved from the Internet <URL:https://www.diva-portal.org/smash/get/diva2:1235486/FULLTEXT01.pdf> [retrieved on 20220701]
- CHENG JI ET AL.: "Flexible Solution-Gated Graphene Field Effect Transistor for Electrophysiological Recording", JOURNAL OF MICROELECTROMECHANICAL SYSTEMS, vol. 23, no. 6, 12 January 2014 (2014-01-12), pages 1311 - 1317, XP011565564, ISSN: 1057-7157
- LUCAS H HESS ET AL.: "Graphene Transistors for Bioelectronics", PROCEEDINGS OF THE IEEE, vol. 101, no. 7, 7 January 2013 (2013-01-07), NEW YORK, US, pages 1780 - 1792, XP011515028, ISSN: 0018-9219

**Description**

**OBJECT OF THE INVENTION**

The object of the invention belongs to the technical field of physics, more precisely to measuring electrical signals.

The object of the invention is aimed to a device and a method using said device, for measuring and recording certain electrophysiological signals.

**BACKGROUND OF THE INVENTION**

There is a great need for flexible, large-scale and high-density arrays with a wide electrophysiological recording bandwidth. Flexible, large-scale and high-density electrode arrays are state-of-the-art. However, those arrays do not provide high-fidelity recordings in the entire frequency bandwidth of electrophysiological signals.

Electrophysiological signals exist in a wide range of frequencies and amplitudes: from minute-long, high-amplitude signals such as cortical spreading depression to millisecond-long microvolt spikes. Recording the full range of electrophysiological signals with high-spatiotemporal resolution would be beneficial to unravel its relation and interactions and to ensure that no meaningful information is lost.

Most microelectrode arrays suffer from voltage drift and oscillations that affect its recording performance of infraslow signals, which frequency is below 0.1 Hz. This is so widely known, that most recording systems include high-pass filters to solve saturation issues that may arise due to baseline drift, at the cost of excluding potential physiological and pathological information from being recorded.

In the last years, there has been a particular resurgence of interest in fluctuations of brain activity at frequencies below 0.1 Hz, commonly referred to as very slow, ultraslow or infraslow activity (ISA). They are suggested to be indicative of brain states (e.g. sleep, anesthesia, coma, wakefulness) and were found to be correlated with resting-state networks in functional magnetic resonance imaging. They also may significantly contribute to the high variability observed in the time course of physiological signals.

There are some reported infraslow signals, such as cortical propagation waves called "cortical spreading depression (CSD)" that are only registered at very low frequencies and therefore it is very difficult to study them in a habitual way due to the impediment of the current electrodes. CSDs are defined as a wave of slow propagation of depolarization of neurons and astrocytes followed by a period of suppression of brain activity and are often triggered when there is a brain episode as in patients who have a vascular or traumatic brain stroke as well as in migraines and other brain pathologies. To monitor or detect them could improve the diagnosis but above all influence on therapeutic changes.

Full-band recordings including infralow frequencies have been traditionally performed with non-invasive techniques such as electroencephalography (EEG) and magnetoencephalography (MEG). However, their limited spatial resolution and averaged signal impose serious limitations; e.g. EEG alone has not yet been sufficient for non-invasive CSD detection. For these reason, invasive electrophysiological techniques are the most commonly used to record infraslow brainwaves.

The proper recording of ISA requires the use of direct-coupled amplifiers and extremely stable and low-impedance invasive electrodes. Traditionally, liquid-filled glass micropipettes are used, which allow only one or few-point measurements. For higher spatial resolution and mapping, non-polarizable silver/silver chloride (Ag/AgCl) electrodes could be used, which prevent charge accumulation at the interface and therefore voltage drift. However, due to the toxicity of silver, the use of such electrodes for human or chronic animal *in vivo* monitoring is not an option. This has fostered the search for alternative microelectrode materials with low impedance and drift although none has yet been found capable of offering comparable performance as Ag/AgCl electrodes. So, ISA recordings in humans are currently performed with platinum electrodes, which challenge CSD detection due to artifacts and transients. Importantly, baseline drift in the form of baseline oscillations in the infralow frequencies, hamper the determination of its "true" characteristics such as amplitude or waveform as any high-pass filter used to remove such effects will alter the signal shape.

Another intrinsic limitation of microelectrode technology is based on the relation between the microelectrode impedance and the input impedance of the recording equipment ( $Z'_e$ and $Z'_a$, respectively).

The recorded signal ($V_{in}$) is determined by the voltage divider formed by both impedances:

$$V_{in}(f) = I(f)Z'_a(f) = \frac{V_{sig}(f)\, Z'_a(f)}{Z'_a(f) + Z'_e(f)} \tag{1}$$

Eq. (1) implies that when $Z'_a$, is not substantially larger than $Z'_e$, the recorded signals will be attenuated and delayed with respect to $V_{sig}$. Even using high-input impedance amplifiers, for 50 µm diameter gold microelectrodes, an attenuation of more than 50% is expected. It is important to highlight that the $Z'_a >> Z'_e$ requirement to achieve a voltage gain equal to 1 is compromised when the electrode area is scaled down, due to the inverse relation between electrode impedance and its area leading to high-pass filtering of the recorded signals.

Therefore, miniaturization of electrode size to achieve higher spatial resolution causes intrinsic high-pass filtering of ISA due to the associated electrode impedance increase.

Invasive optical techniques such as calcium imaging are also used to monitor ISA, but still nowadays have serious challenges to resolve high-frequency activity for a large number of neurons and their intrinsic need of indicators limits the translation to the clinics. Therefore, a technique which allows for measuring large-scale, high-spatiotemporal resolution recordings including infraslow frequencies in a potentially fully implantable, nontoxic, clinical-scale system is still missing.

Alternatively to the commonly used microelectrode technology, recording electrophysiological signals with field-effect transistors (FET) offers several advantages including that they are less sensitive to environmental noise thanks to their intrinsic voltage-to-current amplification, and that they can be easily multiplexed. Nonetheless, the difficulties to combine high gate capacitance and carrier mobility silicon FETs with flexible materials has historically hampered its use for *in vivo* recordings. Graphene solution-gated field-effect transistors (gSGFETs) have been proposed to potentially overcome most previous drawbacks. Graphene flexibility allows gSGFETs to be embedded in ultra-soft and flexible substrates without loss of performance, while its wide electrochemical window and biocompatibility allows direct contact with biological fluids and tissues and ensures a safe operation in *in vivo* conditions. In addition, the two-dimensional nature of graphene provides the highest surface-to-volume ratio possible, making graphene very sensitive to charges at its surface. Importantly, the frequency response of the transconductance of a gSGFET, is flat in a wide bandwidth including inflalow frequencies.

On the other hand ,graphene based solution-gated field effect transistors (G-SgFETs) have been extensively investigated as potential biosensors for various analytes, like in WO2011004136A1 where it is disclosed a sensor for detecting the presence of at least one biological molecule and a method for the production of such a sensor which comprises a patterned graphene structure, at least two electric contacts arranged in contact with the patterned graphene structure for determining a conductivity; and at least one linker attached to at least a portion of the patterned graphene structure, wherein the at least one linker has a binding affinity for the at least one biological molecule.

Blaschke et al., "Mapping brain activity with flexible graphene micro-transistors", 2D Materials, vol. 4, no. 2, IOP Publishing, 2017, according to its abstract, teaches that establishing a reliable communication interface between the brain and electronic devices is of paramount importance for exploiting the full potential of neural prostheses. It is held that current microelectrode technologies for recording electrical activity, however, evidence important shortcomings, e.g. challenging high density integration. Solution-gated field-effect transistors (SGFETs), on the other hand, could overcome these shortcomings if a suitable transistor material were available. Graphene is particularly attractive due to its biocompatibility, chemical stability, flexibility, low intrinsic electronic noise and high charge carrier mobilities. The use of an array of flexible graphene SGFETs is reported for recording spontaneous slow waves, as well as visually evoked and also pre-epileptic activity in vivo in rats. The flexible array of graphene SGFETs shall allow mapping brain electrical activity with excellent signal-to-noise ratio (SNR), suggesting that this technology could lay the foundation for a future generation of in vivo recording implants.

## DESCRIPTION OF THE INVENTION

The invention is as set out in the appended claims.

The present invention addresses the need for flexible, large-scale and high-density arrays with a wide electrophysiological recording bandwidth. The object of the invention is based on a graphene solution-gated field-effect transistor (gSGFETs) preferably an array of graphene solution-gated field-effect transistor (gSGFETs) which are able to record infraslow signals alongside with signals in the typical local field potential bandwidth. The graphene solution-gated field-effect transistor (gSGFETs) are preferably placed on epicortical and intracortical positions.

The present invention simultaneously overcomes the challenges remaining in the prior art by providing vastly increased baseline stability that arises from the electrochemical inertness of graphene, as also surpasses the signal attenuation due to the impedance divisor present in electrode recording systems by using a transistor as a recording element.

The graphene solution-gated field-effect transistor (gSGFETs) of the present invention is fabricated using flexible substrate to overcome the difficulty in conforming to the geometry of different biological structures, hence the graphene solution-gated field-effect transistor (gSGFETs) is preferably flexible. Also, when arranged in an array, said array is designed in an extensible manner such that transistors can be scaled up from micro to macro scale as needed while different kinds of electrical contacts, such as those that sit on the surface of (as oppose to penetrating) tissue.

Hence, the graphene transistor system for measuring electrophysiological signal encompasses a processing unit, and at least one graphene transistor (gSGFET) comprising graphene as channel material contacted by two terminals, to which a tunable voltage source at the drain and source terminals of the transistor (gSGFET) referred to the gate voltage, and

at least one filter (a low-pass filter (LPF) with a gain of $10^4$ configured to generate a low-pass filtered band with a frequency set between 0 Hz and 0.16 Hz or a band-pass filter (BPF) with a gain of $10^6$ configured to generate a band-filtered band with a frequency comprised between 0.16 Hz and 10 kHz) configured to acquire and split a signal from the transistor into at least two frequency bands, a first low frequency band and a second high frequency band, to provide a first low frequency signal and a second high frequency signal are connected; wherein the first low frequency signal and the second high frequency signal are respectively amplified with a gain value.

The method and associated apparatus address the above-mentioned needs in the art by providing an amplification of the signals as also the capability to measure the transistor transfer curve at the site of recording. That allows both choosing the best operation point of the transistor and to apply a calibration methodology (current-to-voltage conversion of the recorded signal) that ensures a high-fidelity recording in a wide-bandwidth.

Of the main applications of the object of the disclosure is that of monitoring full-band brain signals; in either research or clinic implementations like in neurology. These same advantages exist for applications to other biological systems outside of the brain, such as heart, kidneys, stomach, cranial nerves, and other regions. The flexibility and versatility of graphene-transistor arrays allows several applications and deployments which range from subdural, epidural and intracortical devices to other placements in the brain, peripheral and cranial nerves, heart, blood vessels, spinal cord and other biological structures or non-invasive placement similar to an electroencephalogram.

The method for measuring electrophysiological signals according to the invention, using the graphene transistor system can comprise the steps of:

a. splitting an input signal into a first low frequency signal and a second high frequency signal by means of the filter,
b. merging the first low frequency signal and the second high frequency signal weighted by the corresponding gain into a merged signal, and
c. transforming the merged signal into voltage according with the intrinsic gain of the transistor.

The transformation into a voltage signal can be carried out by means of interpolation using the graphene transistor transfer curve $I_{ds}$ - $V_{gs}$.

## DESCRIPTION OF THE DRAWINGS

To complement the description being made and in order to aid towards a better understanding of the characteristics of the disclosure, in accordance with a preferred example of practical embodiment thereof, a set of drawings is attached as an integral part of said description wherein, with illustrative and non-limiting character, the following has been represented:

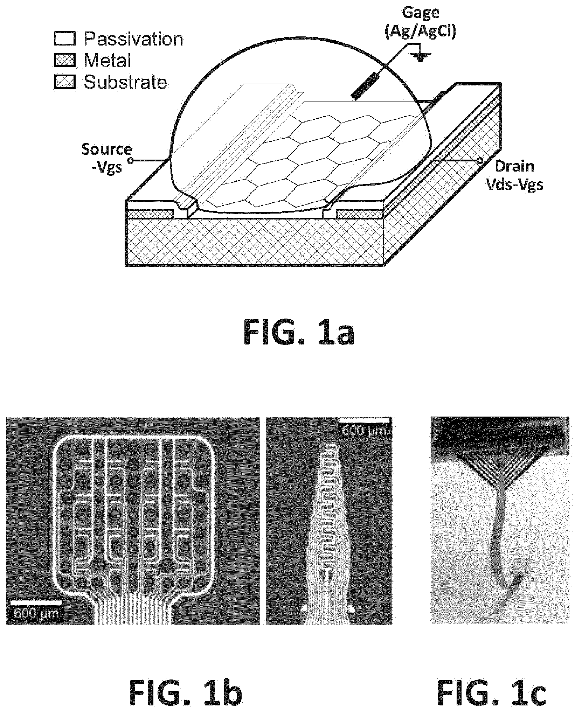

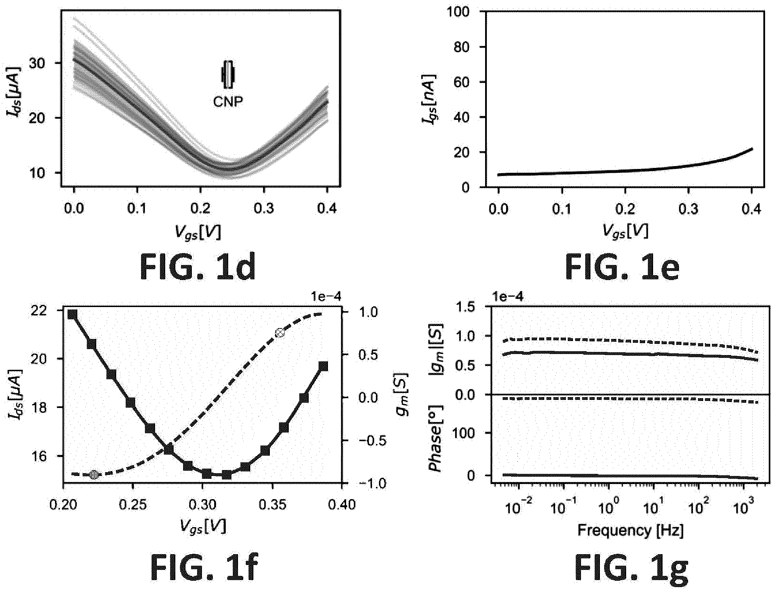

Figures 1a-1g.- Show: a representation of flexible graphene solution-gated field-effect transistor array technology and characterization 1a A schematic of a graphene transistor polarized in common gate mode. 1b: Optical microscope images of the active area of the $4 \times 4$ gSGFET array and the 15 channel intracortical array. 1c: a photograph of the neural probe. 1d: Steady-state characterization of a 100x50-$\mu$m2 gSGFET array in 10 mM phosphate buffered saline (PBS) and with a drain-source voltage bias (Vds) of 50 mV. 1d A graph showing gSGFET transfer curves, drain-source current (Ids) vs gate-source voltage (Vgs), together with the mean (dark curves) and standard deviation (lighter curves). Boxplot inset shows charge neutrality point dispersion (center line, median; box limits, upper and lower quartiles). 1e A graph for the leakage current (Igs) of all gSGFETs in the array. 1f: A graph for the transfer curve (blue squares and line) and its first derivative (transconductance (gm), black line) of a gSGFET. 1g A graph for the frequency response of the transconductance at two different points of the transfer curve (e): Vgs lower than the CNP (green), where gm is negative resulting in a signal inversion (180° phase); and Vgs higher than the CNP (orange), where gm is positive and thus results in no inversion (0° phase). Independently of the branch of the transfer curve where a gSGFET is polarized, the module of gm is similar to the steady-state value for a wide bandwidth ($\approx$ 0 - 1 kHz).

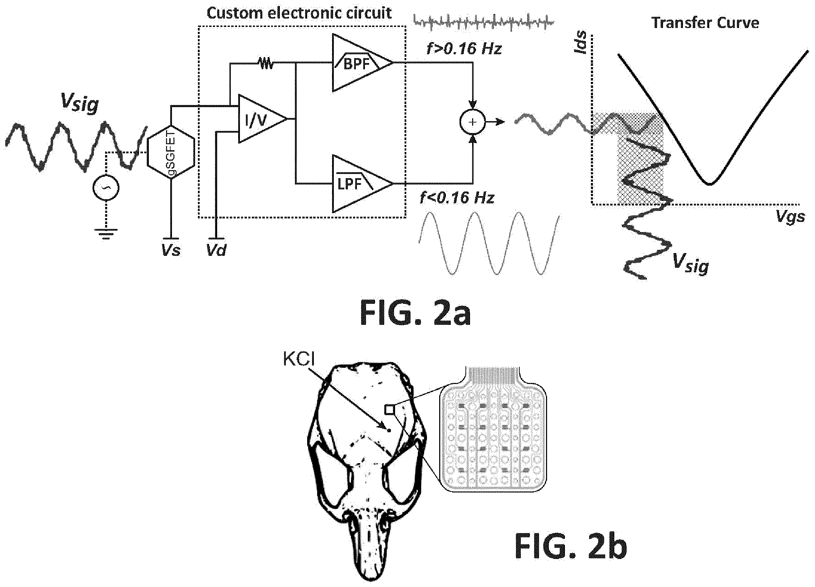

Figures 2a-2d.- Show an exemplary embodiment of the disclosure incorporating a gSGFET, the custom electronic circuit and post-processing methodology as also examples of the recorded signals: infraslow, local field potential, and wide-band *in vivo* gSGFET recordings of neural signals. 2a, Schematic of the gSGFET recording setup and signal post processing methodology. The custom electronic circuit is used to perform the *in vivo* characterization (transfer curve) and record the transistor current in the low-pass-filtered (LPF) band and the band-pass-filtered (BPF) band. From the combination of both signals and taking into account the current-to-voltage conversion, the wide-band signal ($V_{sig}$) is obtained. 2b, Electrophysiological recordings obtained with a gSGFET epicortical array during the induction of four CSD events (blue shade). From top to bottom: current LPF signal, current BPF and voltage-converted wide-band signal.

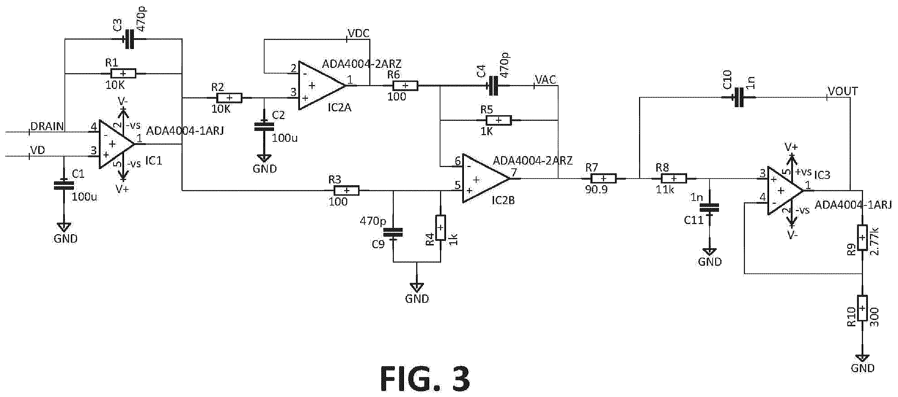

Figure 3.- Shows an exemplary embodiment of the custom electronic circuit. a, Schematic of the custom electronic instrumentation which controls the polarization of the gSGFETS ($V_{gs}$, $V_{ds}$) and amplifies differently the two previously mentioned bands: LPF ($\approx$0-0.16 Hz, gain=$10^4$) and BPF (0.16 Hz-10 kHz, gain=$10^6$). We use the custom electronic instrumentation to characterize the steady-state behaviour of the gSGFETs as well as the AC modulation of graphene transistors.

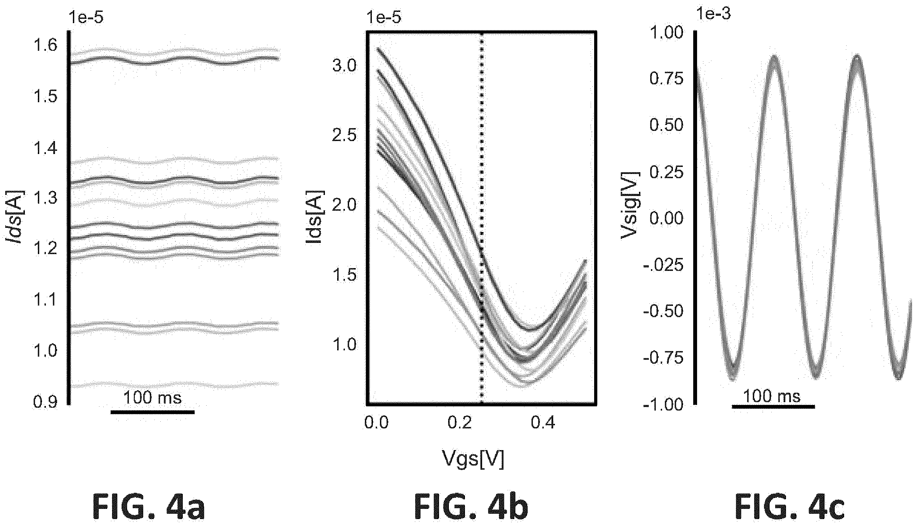

Figure 4.- Shows the calibration procedure of gSGFET current recordings to recover the voltage signal at the gate. a, gSGFET current recordings of a 10 Hz, 0.85 mV-peak sinusoidal gate signal applied through a reference electrode. Graphene transistors are biased at $V_{ds}$=50mV and $V_{gs}$=250mV. b, Transfer curves of the same graphene transistors at $V_{ds}$=50mV. Dotted line indicates the $V_{gs}$ bias voltage used in a. c, Voltage signal as obtained by interpolation of the current signal (a) of each transistor into its corresponding transfer curve and removal of the $V_{gs}$ offset.

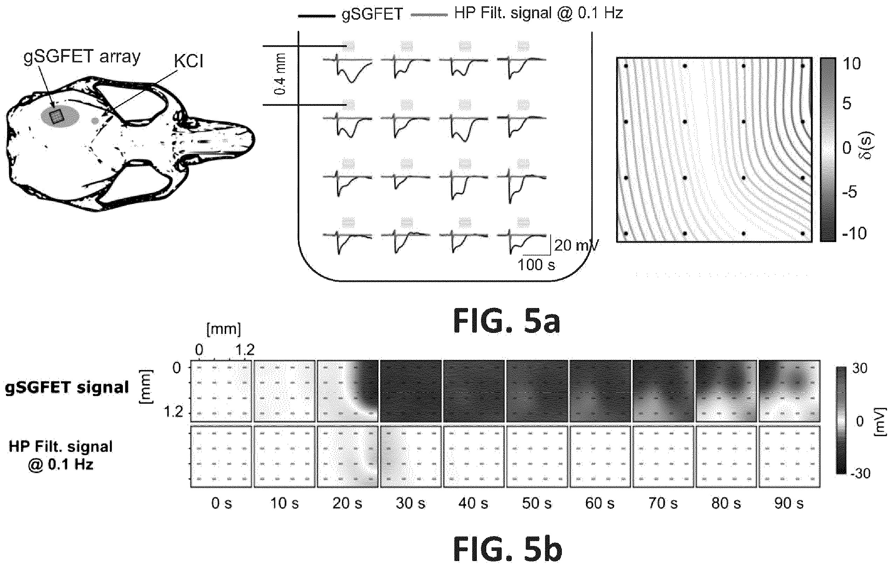

Figure 5.- Shows the mapping cortical spreading depression with graphene transistors. a, Infralow frequency signals recorded by a 4x4, 400 $\mu$m grid spacing, gSGFET array (black lines) during the occurrence of a CSD event as illustrated in the top left schematic. The contour plot shows the time delays of the onset of CSD with respect to the mean time illustrating the spatiotemporal course of the CSD. b, Interpolated spatial voltage maps showing the propagation of the same CSD event as measured by the gSGFET array. a,b High pass filtered recordings at 0.1Hz (red lines in a and bottom spatial voltage maps in b) are included to illustrate the loss of signal information in conventional microelectrode recordings.

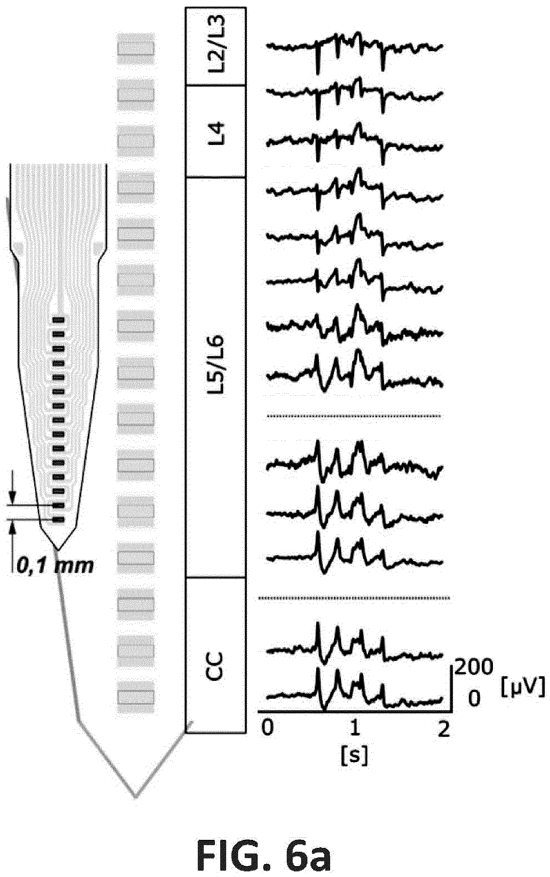

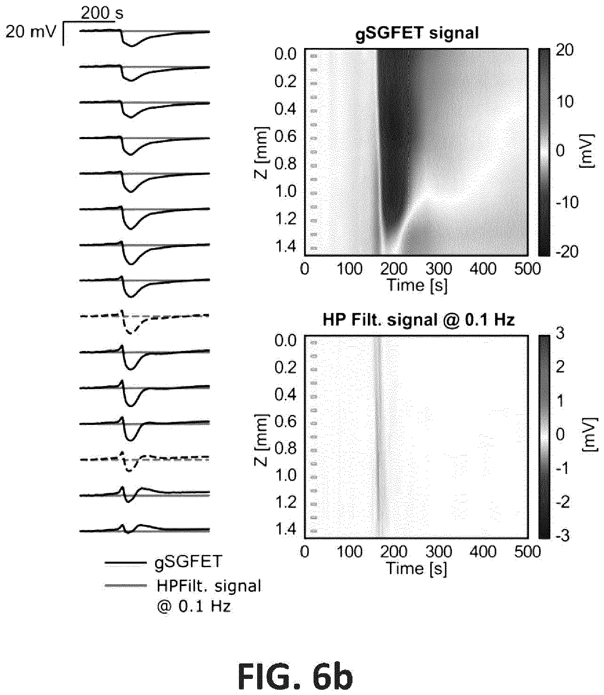

Figures 6a, 6b.- Depict the depth profile of the infralow-frequency voltage variations induced by cortical spreading depression in a rat cortex. a, Layout of the fabricated 15-channel graphene intracortical probe and ordered local field potential recordings. Infralow-frequency recordings (black lines) during the occurrence of a CSD event. Dashed lines, have been interpolated from nearby transistors. b, Colour maps of the temporal course of the infraslow changes during a CSD event across the depth of a rat cortex. a-b, Same signal high-pass filtered at 0.1 Hz (red lines) and their spatiotemporal colour map are included to illustrate the loss of information in conventional microelectrode recordings.

## PREFERRED EMBODIMENT OF THE DISCLOSURE

A first aspect of the disclosure is aimed to a system for registering electrophysiological infraslow signals like cortical spreading depression (CSD) signals i.e. those with a frequency value below 0.1Hz; the device comprising a processing unit associated or embedded in the very device, and at least one graphene transistor (gSGFET), preferably an array of graphene transistors, comprising graphene as channel material contacted by source and drain terminals, with a reference as gate terminal. Said graphene transistor is connected to at least a filter like a low pass filter (LPF). The recorded current signal is transformed into a voltage signal using the transistor transfer curve $I_{ds}$ - $V_{gs}$ recorded to the start of the recordings.

In an alternative embodiment of the disclosure, at least one band pass filter (BPF) is arranged in either a sequential or a cascade arrangement along with the low pass filter (LPF), but both filters (LPF, BPF) being configured so that the respective cutting points have the same value.

An gSGFETS is a device in which graphene is used as channel material, contacted by two metal leads (source and drain terminals), and is immersed in an electrolyte solution where a reference electrode is used as gate terminal (Fig. 1a). Flexible probes containing arrays of gSGFETs in both epicortical and intracortical designs were produced. In particular, a 4x4 array of 100 $\mu$m wide by 50 $\mu$m long graphene channels were designed for epicortical recordings while a design consisting of a linear array of 15 graphene channels (80 $\mu$m width, 30 $\mu$m length) was used for intracortical recordings (Fig. 1b). Both array designs were fabricated on a 10 $\mu$m thick polyimide layer coated on a 4-inch silicon wafer. Flexible gSGFET arrays were placed in zero insertion force connectors for interfacing with recording electronics (Fig. 1c). The transfer curve, drain current (I_ds) vs gate-source voltage (V_gs), of all gSGFETs in each array was measured with a fixed drain-source voltage (V_ds). The dispersion of the charge neutrality point (CNP=243.6 $\pm$ 6.1 mV), which is the minimum of the transfer curve, indicates the homogeneity of the transistors (Fig. 1d). Importantly, since the V_gs and V_ds bias are shared, the small CNP dispersion allows near-optimal recording performance for all gSGFETs in the same array. Figure 1e shows the sum of leakage current (I_gs) for all gSGFETs in the array, which is in the nA range throughout the voltage sweep, demonstrating the good insulation of the passivation layer and the negligible reactivity of the graphene. Furthermore, we measured the frequency response of the transconductance (gm) of a gSGFET, which indicates the efficiency of the signal coupling (($\partial$I_ds)($\partial$V_gs )), obtaining constant values in a wide bandwidth including inflalow frequencies (Fig. 1f-g). The negative gm for Vgs values lower than the CNP results in an inversion (180° phase) of the signals measured at such bias; for Vgs values higher than the CNP the signal phase is preserved.

The device of the disclosure was compared to conventional high-pass filtered recordings, to do so the propagation of cortical spreading depression (CSD) events using a 4x4 epicortical gSGFET array was mapped and then compared with what is observed in conventional high-pass filtered recordings (Fig. 5a-b). The recording of the whole CSD event with the gSGFET array reveals that while the onset of the negative shift is similar for all gSGFETs, there is much more variety in the subsequent recovery, with some transistors exhibiting a second negative shift with higher amplitude than the first one. This effect can also be observed in the last frames (corresponding to 80 s and 90 s) of the spatial maps of gSGFET recordings (Fig. 5b) where recovered and still depressed brain areas coexist. Importantly, this information is lost in conventional microelectrode recordings, where only the CSD onset is observed due to the high pass filter in the recording electronics. The following results are referred to a sample of 10 CSDs collected from two different subjects in the somatosensory cortex: we found that the mean duration of CSD events is $47.24 \pm 7.65$ s and a speed of propagation of $7.68 \pm 1.35$ mm/min, in agreement with the literature defining CSDs as infraslow brainwaves.

To further illustrate the potential of the device of the disclsoure and taking advantage of the design versatility offered by this technology, a linear array of 15 gSGFETs spanning the entire depth of the cortex (Fig. 6a) was arranged. From either the ordered recording or the spatiotemporal voltage map (Fig. 6b), it can be seen how CSD occurs in the whole cortex depth. These results highlight the capability of the device of the disclosure to reveal the rich pattern of infraslow signals in the cortex; in this particular case, a transition from a superficial long depolarization to a shorter one preceded and followed by a hyperpolarization in the deeper layers is clearly observed. The origin of such depth-dependent effect is not well understood and will be the target of further investigations, taking advantage of the demonstrated capability of gSGFET technology to monitor ISA with high spatial resolution.

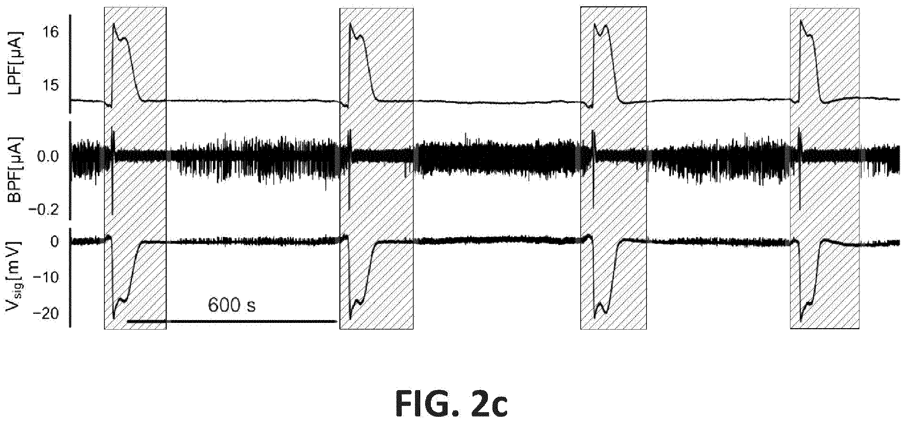

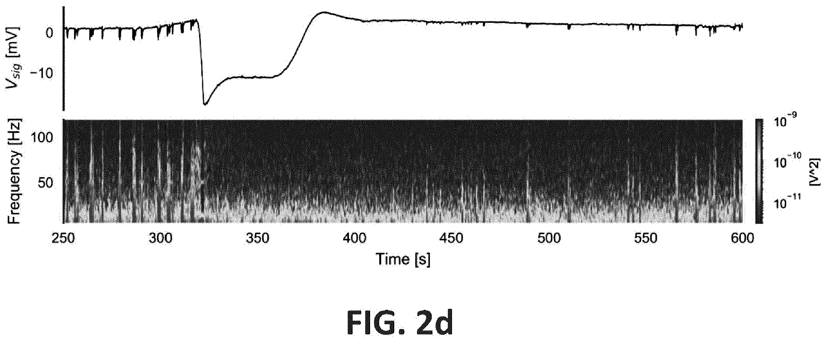

In a second aspect of the disclosure a method for recording infraslow brain signals, being infraslow signals those with a frequency value below 0.1Hz is provided, Cortical spreading depression (CSD) was chosen to illustrate the capabilities of the object of the disclosure to record in a wide bandwidth. Experimentally, two craniotomies were performed over the left hemisphere of isoflurane-anaesthetized Wistar rats: a larger craniotomy over the primary somatosensory cortex, where the epicortical probe was placed, and a smaller one in the frontal cortex, where 5 mM KCl was applied locally to induce CSD (Fig. 2b). A custom electronic circuit allowed us to simultaneously record at two frequency bands: low-pass filtered band (LPF, $\approx$0-0.16 Hz) and band-pass filtered band (BPF, 0.16 Hz-10 kHz) with different gains ($10^4$, and $10^6$ respectively) to avoid amplifier saturation due to the high-amplitude CSD signal. In a first set of experiments, we recorded the LPF and BPF current signals with an epicortical gSGFET array during the induction of CSD events (Fig. 2c). The graphene transistors were polarized in the hole conduction regime, i.e. $V_{gs}$ < CNP (negative $g_m$); therefore, the recorded LPF and BPF current signals are inverted with respect to the voltage signal occurring at the gate. The LPF signal shows the very slow CSD event whereas the BPF signal corresponds to the local field potential, revealing the silencing of activity typical of cortical spreading depression. After summation of the LPF and BPF signals and then transforming the current into a voltage signal (using the transistor transfer curve $I_{ds}$ - $V_{gs}$ recorded *in vivo* prior to the start of the recordings), the wide-band electrophysiological signal can be obtained (see Fig. 2 a, c). In each CSD event a small positive shift of 1-2 mV generally precedes the depression, immediately after which a steep negative change ($\approx$ -20 mV) can be observed, which slowly recovers during the next minute or so. The CSD-associated silencing of high-frequency activity and its progressive recovery is shown in the voltage wave and spectrogram of Fig. 2d.

## Claims

1. Graphene transistor system for measuring electrophysiological signals, comprising:

   a. a processing unit, and
   b. at least one graphene transistor (gSGFET) comprising graphene as channel material contacted by two terminals,

   being the device **characterized by** comprising connected to the graphene transistor (gSGFET):

   a. a tunable voltage source connected at the drain and source terminals of the graphene transistor (gSGFET) referred to the gate voltage, and
   b. at least one filter configured to acquire and split a signal from the graphene transistor into at least two frequency bands, a low frequency band and a high frequency band, to provide a first low frequency signal and a second high frequency signal, wherein the first low frequency signal and the second high frequency signal are respectively amplified with a gain value.

2. Graphene transistor system for measuring electrophysiological according to claim 1 wherein the filter is configured to generate:

   a. a low-pass filtered band with a frequency set between 0Hz and 0.16 Hz, and
   b. a band-filtered band with a frequency comprised between 0.16 Hz and10 kHz.

3. Graphene transistor system for measuring full-band electrophysiological according to claim 2 wherein the low-pass filter (LPF) and the band-pass filter (BPF) have different gains; $10^4$ and $10^6$ respectively.

4. Method for measuring electrophysiological signals, using the graphene transistor system of any one of claims 1-3, the method being **characterized by** comprising:

   a. splitting an input signal into a first low frequency signal and a second high frequency signal by means of the filter,
   b. merging the first low frequency signal and the second high frequency signal weighted by the corresponding gain into a merged signal, and
   c. transforming the merged signal into voltage according with the intrinsic gain of the graphene transistor.

5. Method according to claim 4, wherein the gain value of the amplification is different for each signal of the first low frequency signal and the second high frequency signal.

6. Method according to claim 4, wherein the transformation into a voltage signal is carried out by means of interpolation using a graphene transistor transfer curve $I_{ds}$ - $V_{gs}$ of the graphene transistor, wherein $I_{ds}$ is a drain-source current of the graphene transistor and $V_{gs}$ is a gate-source voltage of the graphene transistor.

7. Method according to claim 6, wherein the graphene transistor transfer curve $I_{ds}$ - $V_{gs}$ is generated with a fixed drain-source voltage (V_ds) of the graphene transistor.


**Patentansprüche**

1. Graphen-Transistor-System zur Messung elektrophysiologischer Signale, aufweisend:

   a. eine Verarbeitungseinheit, und
   b. mindestens einen Graphen-Transistor (gSGFET) mit Graphen als Kanalmaterial und von zwei Anschlüssen kontaktiert,

   wobei die Vorrichtung **dadurch gekennzeichnet ist, dass** sie mit dem Graphen-Transistor (gSGFET) verbunden aufweist:

   a. eine abstimmbare Spannungsquelle, die an die Drain- und Source-Anschlüsse des Graphen-Transistors (gSGFET) in Bezug auf die Gate-Spannung angeschlossen ist, , und
   b. mindestens einen Filter, der dazu eingerichtet ist, ein Signal von dem Graphen-Transistor zu erfassten und in mindestens zwei Frequenzbänder, ein niederfrequentes Band und ein hochfrequentes Band, aufzuteilen, um ein erstes niederfrequentes Signal und ein zweites hochfrequentes Signal bereitzustellen, wobei das erste niederfrequente Signal und das zweite hochfrequente Signal jeweils mit einem Verstärkungswert verstärkt werden.

2. Graphen-Transistor-System zur Messung elektrophysiologischer Signale nach Anspruch 1, wobei der Filter dazu eingerichtet ist, Folgendes zu erzeugen:

   a. ein tiefpassgefiltertes Band mit einer Frequenz zwischen 0 Hz und 0,16 Hz, und
   b. ein bandgefiltertes Band mit einer Frequenz zwischen 0,16 Hz und 10 kHz.

3. Graphen-Transistor-System zur Messung elektrophysiologischer Vollbandsignale nach Anspruch 2, wobei der Tiefpassfilter (LPF) und der Bandpassfilter (BPF) unterschiedliche Verstärkungen aufweisen; $10^4$bzw. $10^6$.

4. Verfahren zur Messung elektrophysiologischer Signale unter Verwendung des Graphen-Transistor-Systems nach einem der Ansprüche 1 bis 3, wobei das Verfahren **dadurch gekennzeichnet ist, dass** es aufweist:

a. Aufteilen eines Eingangssignals in ein erstes niederfrequentes Signal und ein zweites hochfrequentes Signal durch den Filter,

b. Zusammenführen des ersten niederfrequenten Signals und des zweiten hochfrequenten Signals, gewichtet mit der korrespondierenden Verstärkung, zu einem zusammengeführten Signal, und

c. Umwanden des zusammengeführten Signals in eine Spannung gemäß der intrinsischen Verstärkung des Graphen-Transistors.

5. Verfahren nach Anspruch 4, wobei der Verstärkungswert der Verstärkung für jedes Signal von dem ersten niederfrequenten Signal und dem zweiten hochfrequenten Signal unterschiedlich ist.

6. Verfahren nach Anspruch 4, wobei das Umwandeln in ein Spannungssignal mittels Interpolation unter Verwendung einer Graphen-Transistor-Übertragungskurve $I_{ds}$ - $V_{gs}$ des Graphen-Transistors durchgeführt wird, wobei $I_{ds}$ ein Drain-Source-Strom des Graphen-Transistors und $V_{gs}$ eine Gate-Source-Spannung des Graphen-Transistors ist.

7. Verfahren nach Anspruch 6, wobei die Graphen-Transistor-Übertragungskurve $I_{ds}$ - $V_{gs}$ mit einer fixen Drain-Source-Spannung (V_ds) des Graphen-Transistors erzeugt wird.

**Revendications**

1. Système à transistors en graphène destiné à mesurer des signaux électrophysiologiques, comprenant :

   a. une unité de traitement, et
   b. au moins un transistor en graphène (gSGFET) comprenant du graphène comme matériau de canal en contact avec deux bornes,

   étant le dispositif **caractérisé par le fait qu'**il comprend, connectés au transistor en graphène (gSGFET) :

   a. une source de tension accordable connectée aux bornes de drain et de source du transistor en graphène (gSGFET) référencée à la tension de grille, et
   b. au moins un filtre configuré pour acquérir et diviser un signal provenant du transistor en graphène en au moins deux bandes de fréquence, une bande basse fréquence et une bande haute fréquence, afin de fournir un premier signal basse fréquence et un second signal haute fréquence, dans lequel le premier signal basse fréquence et le second signal haute fréquence sont respectivement amplifiés avec une valeur de gain.

2. Système à transistors en graphène destiné à mesurer des signaux électrophysiologiques selon la revendication 1, dans lequel le filtre est configuré pour générer :

   a. une bande filtrée par filtre passe-bas dont la fréquence est comprise entre 0 Hz et 0,16 Hz, et
   b. une bande filtrée par bande dont la fréquence est comprise entre 0,16 Hz et 10 kHz.

3. Système à transistors en graphène destiné à mesurer des signaux électrophysiologiques à bande complète selon la revendication 2, dans lequel le filtre passe-bas (LPF) et le filtre passe-bande (BPF) ont des gains différents ; $10^4$ et $10^6$ respectivement.

4. Procédé destiné à mesurer des signaux électrophysiologiques, à l'aide du système à transistors en graphène selon l'une quelconque des revendications 1 à 3, le procédé étant **caractérisé par le fait qu'**il comprend :

   a. la division d'un signal d'entrée en un premier signal basse fréquence et en un second signal haute fréquence au moyen du filtre,
   b. la fusion du premier signal basse fréquence et du second signal haute fréquence pondérés par le gain correspondant en un signal fusionné, et
   c. la transformation du signal fusionné en tension selon le gain intrinsèque du transistor en graphène.

5. Procédé selon la revendication 4, dans lequel la valeur de gain de l'amplification est différente pour chaque signal parmi le premier signal basse fréquence et le second signal haute fréquence.

6. Procédé selon la revendication 4, dans lequel la transformation en un signal de tension est réalisée au moyen d'une interpolation à l'aide d'une courbe de transfert de transistor en graphène $I_{ds}$ - $V_{gs}$, dans lequel $I_{ds}$ est un courant drain-source du transistor en graphène et $V_{gs}$ est une tension grille-source du transistor en graphène.

7. Procédé selon la revendication 6, dans lequel la courbe de transfert de transistor en graphène $I_{ds}$ - $V_{gs}$ est générée avec une tension drain-source (V_ds) fixe du transistor en graphène.

FIG. 1a

FIG. 1b

FIG. 1c

FIG. 1e

FIG. 1g

FIG. 1d

FIG. 1f

FIG. 2a

FIG. 2b

FIG. 2c

**FIG. 2d**

FIG. 3

**FIG. 4a**

**FIG. 4b**

**FIG. 4c**

EP 3 879 582 B1

**FIG. 5a**

**FIG. 5b**

**FIG. 6a**

FIG. 6b

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011004136 A1

**Non-patent literature cited in the description**

- Mapping brain activity with flexible graphene micro-transistors. **BLASCHKE et al.** 2D Materials. IOP Publishing, 2017, vol. 4